# DEMANDE DE BREVET EUROPEEN

(11) **EP 1 915 948 A2**
(43) Date de publication de la demande: **30.04.2008**
(21) Numéro de dépôt: 07118497.2
(22) Date de dépôt: 15.10.2007
(51) Int. Cl.: A61B 6/04, A61G 7/05, A47C 31/10

(54) **HOUSSE DE PROTECTION D'UN ELEMENT DE SUPPORT DU TYPE MATELAS, COMPRENANT UNE POCHE APTE A CONTENIR UNE CASSETTE DE RADIOGRAPHIE ET PROCEDE DE RADIOGRAPHIE**

(30) Priorité: 23.10.2006 FR 0654447
(71) Demandeur: HILL-ROM INDUSTRIES S.A., 34100 Montpellier (FR)
(72) Inventeur: Caminade, Jean Luc, 34430, SAINT JEAN DE VEDAS (FR); Flocard, Thierry, 34000, MONTPELLIER (FR)
(74) Mandataire: Domange, Maxime

(57) **Abrégé**

La présente invention concerne une housse de protection (1) d'un élément de support principal (2) du type coussin ou matelas, sur laquelle une partie au moins du corps d'un patient (7) à radiographier est destinée à reposer, ladite housse comprenant au moins une partie supérieure (1₁) sensiblement plane lorsqu'elle est mise en place de manière à recouvrir la surface supérieure (2₁) dudit élément de support principal et des rebords latéraux (1₂), aptes à recouvrir en partie au moins les flancs (2₂) de l'élément de support principal (2), caractérisée en ce qu'elle comprend :
- une poche interne (3) souple, s'étendant dessous ladite partie supérieure (1₁) de la housse, apte à contenir une cassette de radiographie (5), ladite poche comprenant au moins une ouverture (3a) permettant l'introduction d'une dite cassette, l'extrémité de la poche formant ouverture débouchant sur et étant fixée à au moins un dit rebord latéral (1₂) de la housse, à proximité du bord longitudinal (1₃) de ladite partie principale de housse, et
- un élément de support secondaire (4) de type matelas ou coussin, moins épais que ledit élément de support principal (2), de préférence d'épaisseur inférieure à 5 cm, solidaire de ladite housse, intercalé entre ladite poche (3) et ladite partie supérieure (1₁) de housse et recouvrant au moins ladite poche.

## Description

La présente invention concerne un procédé et dispositif de radiographie à l'aide d'une cassette de radiographie adapté sur un élément de support, de type coussin ou matelas disposé respectivement sur un siège ou lit, sur lequel repose une partie au moins du corps du patient, permettant de radiographier ledit patient installé sur ledit élément de support.

Une cassette de radiographie est classiquement constituée d'une plaque à cadre parallélépipédique de faible épaisseur rigide tel qu'un cadre en métal ou en matériau synthétique incorporant un support photosensible d'un type de rayonnement mis en oeuvre, notamment le plus généralement aux rayons X.

On connaît un procédé qui consiste à soulever le patient et placer la cassette de radiographie directement sous la partie du corps du patient à radiographier entre le corps du patient et la face de l'élément de support supportant la partie du corps à radiographier, notamment la face supérieure de l'élément de support dans le cas d'un matelas sur un lit.

Ce procédé est peu pratique car il nécessite le soulèvement du patient et donne lieu à un dérangement du patient par plusieurs manipulateurs. En outre, dans le cas de patients fortement traumatisés, ce type de manipulation est proscrite car toute manipulation du patient constitue un risque pour son intégrité, notamment en cas de fractures du rachis dorsal ou de brûlures importantes.

On connaît aussi un procédé dans lequel la cassette de radiographie coopère avec un support de cassette solidaire du châssis de lit ou de siège, en fait dessous ou derrière celui-ci, lequel est ensuite recouvert dudit élément de support de type coussin ou matelas à des fins de confort du patient dont la partie du corps à radiographier repose sur ledit élément de support.

Ce procédé est moins dérangeant pour le patient mais pose un problème de qualité de l'image obtenue par radiographie, compte-tenu de l'éloignement des zones du corps à radiographier, tant de la hauteur du châssis et de l'épaisseur de l'élément de support.

Un inconvénient de ce procédé est en particulier que le matériel disposé entre la cassette et le corps à radiographier peut générer des artéfacts.

Le but de la présente invention est de fournir des procédé et dispositif améliorés permettant de radiographier, à l'aide d'une cassette, une partie au moins du corps d'un patient reposant sur un élément de support du corps, tel qu'un coussin ou matelas, disposés respectivement sur un siège ou un lit, permettant de radiographier au plus près la partie du corps du patient tout en préservant le patient sans le blesser, c'est-à-dire en limitant au maximum toute manipulation du patient et avec un maximum de confort pour celui-ci lors de la manipulation puis lors de la radiographie après introduction de la cassette.

Un but de la présente invention est donc de fournir des procédé et dispositif plus simples à mettre en oeuvre et plus performants, notamment en terme de diminution des risques de blessure du patient et d'amélioration de la qualité radiographique et donc fournir un procédé et dispositif facilitant les manipulations pour l'introduction et le retrait de la cassette.

Pour ce faire, la présente invention fournit une housse de protection d'un élément de support principal du type coussin ou matelas, sur laquelle une partie au moins du corps d'un patient à radiographier est destinée à reposer, ladite housse comprenant au moins une partie supérieure sensiblement plane lorsqu'elle est mise en place de manière à recouvrir la surface supérieure dudit élément de support principal et des rebords latéraux, aptes à recouvrir en partie au moins les flancs de l'élément de support principal, caractérisée en ce qu'elle comprend :
- une poche interne souple, s'étendant dessous ladite partie supérieure de la housse, apte à contenir une cassette de radiographie, ladite poche comprenant au moins une ouverture permettant l'introduction d'une dite cassette, l'extrémité de la poche formant ouverture débouchant sur et étant fixée à au moins un dit rebord latéral de la housse, à proximité du bord longitudinal de ladite partie principale de housse, et
- un élément de support secondaire de type matelas ou coussin, moins épais que ledit élément de support principal, de préférence d'épaisseur inférieure à 5 cm, solidaire de ladite housse, intercalé entre ladite poche et ladite partie supérieure de housse et recouvrant au moins ladite poche.

La mise en oeuvre dudit élément de support secondaire d'une part, protège le patient lors de l'introduction de ladite cassette dans ladite poche et en cours de radiographie et, d'autre part, facilite la manipulation d'introduction de ladite cassette par un seul opérateur, en effaçant les déformations de surface naturelles du corps physique, lesquelles sont particulièrement accentuées dans le cas de patients décharnés, dont les épines osseuses sont saillantes ou encore dans le cas de patients présentant une spasticité.

La poche contribue à protéger les éléments de support principal et secondaire lors des manipulations de la cassette en contact avec ceux-ci pour l'introduction et le retrait de la cassette et facilite la mise en place de la cassette, à savoir son guidage et sont maintient en positionnement correct, entre la housse et l'élément de support principal.

De préférence, ledit élément de support principal et ledit élément de support secondaire sont constitués par des cellules en forme de boudins disposés transversalement remplies d'un fluide, de préférence de l'air, les cellules dudit élément de support secondaire présentant une section réduite par rapport à celle dudit élément de support principal, de préférence ladite section réduite présentant une plus grande dimension, notamment un diamètre inférieur à 5 cm.

De préférence encore, les cellules desdits élément de support principal et élément de support secondaire sont remplies d'air et sont connectées à un dispositif de régulation de la pression à l'intérieur desdites cellules, en fonction de la portance du patient au niveau de différentes zones du corps du patient, la pression à l'intérieur des cellules dudit élément de support secondaire étant sensiblement identique à celle des cellules de la zone dudit élément de support principal en vis-à-vis.

Ces matelas constitués de cellules en forme de boudins ou cylindres transversaux gonflés d'air ont une visée thérapeutique, notamment dans la prévention d'escarres.

En effet, ils permettent de réguler la portance des patients de façon connue en variant et régulant la pression d'air des différentes cellules dans les différentes zones du matelas selon leur localisation pour obtenir une distribution optimale de la pression d'interface entre le corps et les matelas, ce qui permet, le cas échéant, de régler la pression dudit élément de support secondaire et dudit élément de support principal, notamment de la zone de l'élément de support principal situé en vis-à-vis dudit élément de support secondaire, d'une part, pour faciliter l'introduction et l'évacuation de la cassette de radiographie, et d'autre part, pour continuer la régulation de la pression d'interface, notamment pour homogénéiser la pression d'interface entre le patient et lesdits matelas pendant la radiographie.

Dans un mode préféré de réalisation, ladite poche est constituée d'un tissu synthétique fin présentant des propriétés de résistance mécanique à la déchirure ainsi qu'un coefficient de glissement élevé. En effet, en poussant la cassette de radiographie dans la poche, celle-ci ne doit pas être freinée par l'adhérence du tissu sur sa face interne à ladite poche d'une part et, d'autre part, les parties saillantes aux angles de la cassette ne doivent pas risquer de déchirer le tissu.

Plus particulièrement, ladite poche est constituée d'un tissu synthétique d'un grammage inférieur à 100g/m², calandré à chaud et dont au moins une face correspondant à la face interne de la poche est revêtue d'une couche de résine polymère notamment un polymère fluoré, lui conférant des propriétés de glissement.

Ce type de calandrage à chaud écrase les fils synthétiques en surface et ferme le réseau du tissu, ce qui confère au matériau des propriétés mécaniques de résistance à la traction et résistance à la déchirure amorcée élevée ainsi qu'un fort coefficient de glissement.

Des tissus de ce type sont utilisés pour confectionner des toiles de parachute et des spinnackers de voiliers.

Plus particulièrement, la poche est constituée d'un tissu présentant une résistance à la traction d'au moins 20 daN, de préférence au moins 40 daN selon le test de la norme ISO 13934-1 et une résistance à la déchirure amorcée d'au moins 10N, de préférence au moins 20N selon le test ISO 13937-1.

Ce test de résistance à la traction signifie que le tissu résiste à une traction allant jusqu'à 20 daN, de préférence jusqu'à 40 daN et ne se déchire pas plus lorsqu'il y a une déchirure amorcée sous l'effet d'une traction d'au moins 10N, de préférence au moins 20N.

Ces propriétés de résistance mécanique et de glissement du tissu constitutif de la poche sur la surface interne à la poche, facilitent les opérations d'introduction ou de retrait de la cassette par un opérateur unique sans dérangement excessif du patient à radiographier.

Dans un mode de réalisation particulier, une partie supérieure de l'extrémité de la poche formant l'ouverture est fixée à un dit bord longitudinal de ladite partie supérieure de housse, et l'autre partie inférieure de l'extrémité de la poche formant ladite ouverture est fixée en haut dudit rebord latéral correspondant de la housse.

Avantageusement, ladite poche comporte deux dites ouvertures opposées, ladite poche formant ainsi une gaine traversant la housse entre les deux dits bords longitudinaux opposés et rebords latéraux opposés de ladite housse.

Cette gaine constitue une manche permettant le passage de la cassette ou l'introduction de la cassette par une quelconque des deux ouvertures.

En outre, en cas de radiographies successives, ce mode de réalisation permet d'enchaîner les radiographies plus rapidement et plus facilement par un mouvement traversant une ligne unidirectionnelle, en insérant la ou les cassettes, le cas échéant, les unes à la suite des autres par une même ouverture et en les évacuant par l'ouverture opposée, après radiographie.

De préférence, ladite ouverture de poche est équipée d'un dispositif d'ouverture/fermeture réversible à glissière, de préférence protégé par un rabat latéral solidaire de ladite housse.

Ce dispositif d'ouverture/fermeture réversible et ce rabat permettent de protéger l'intérieur de la housse de pénétration de liquide et/ou toute pollution ou contamination générée par le patient et/ou tout élément parasite risquant de générer des artéfacts sur la radiographie.

Dans un mode de réalisation particulier, la dimension de la poche dans la direction longitudinale dudit élément de support principal permet de positionner une dite cassette en vis-à-vis d'une zone de ladite partie supérieure de la housse sur laquelle est destinée à reposer une partie du corps du patient s'étendant depuis l'occiput jusqu'au bassin du patient, de préférence sur une distance de ½ à 2/3 de la longueur de ladite housse depuis une de ces extrémités dans la direction longitudinale.

La présente invention a également pour objet une housse de protection selon l'invention, caractérisée en ce qu'elle recouvre un dit élément de support principal de type coussin ou matelas, de préférence disposé respectivement sur un siège ou un lit.

La présente invention a également pour objet un procédé de radiographie d'une partie au moins du corps d'un patient à l'aide d'une cassette de radiographie, au moins la partie du corps du patient à radiographier appuyant sur un élément de support principal du type coussin ou matelas, caractérisé en ce que l'on insert une dite cassette dans une poche d'une housse de protection selon l'invention.

Dans un mode de réalisation avantageux du procédé selon l'invention, on met en oeuvre une housse comprenant une poche tubulaire en forme de gaine avec deux ouvertures débouchant et fixées sur les bords longitudinaux opposés et rebords latéraux opposés d'une dite housse et on introduit une dite cassette par une dite ouverture, on réalise une radiographie et on évacue ladite cassette par l'ouverture opposée.

Dans un mode préféré de réalisation du procédé selon l'invention, on régule la pression à l'intérieur desdits élément de support principal et élément de support secondaire constitués de cellules en forme de boudins disposés transversalement et remplis d'air, de façon à homogénéiser la pression d'interface entre le corps du patient et lesdits éléments de support principal et secondaire, en maintenant une pression à l'intérieur des cellules dudit élément de support secondaire sensiblement identique aux cellules dans la zone dudit élément de support principal en vis-à-vis dudit élément de support secondaire.

D'autres caractéristiques et avantages de la présente invention apparaîtront à la lumière de la description détaillée d'un mode de réalisation qui va suivre, faîte en référence aux figures 1 à 3, dans lesquelles :
- La figure 1 représente une vue d'ensemble, présentant une housse de protection 1 selon l'invention recouvrant un matelas principal 2 avec une cassette de radiographie 5 en cours d'insertion dans une poche 3 selon l'invention ; et
- La figure 2 est une vue en coupe longitudinale de l'ensemble selon la figure 1 avec un patient allongé 8 sur une housse selon l'invention ; et
- La figure 3 est une vue en coupe transversale selon AA de la figure 2.
- La figure 4 est une vue en coupe transversale desdits éléments de support principal et secondaire constitués de boudins relevés en inclinaison.

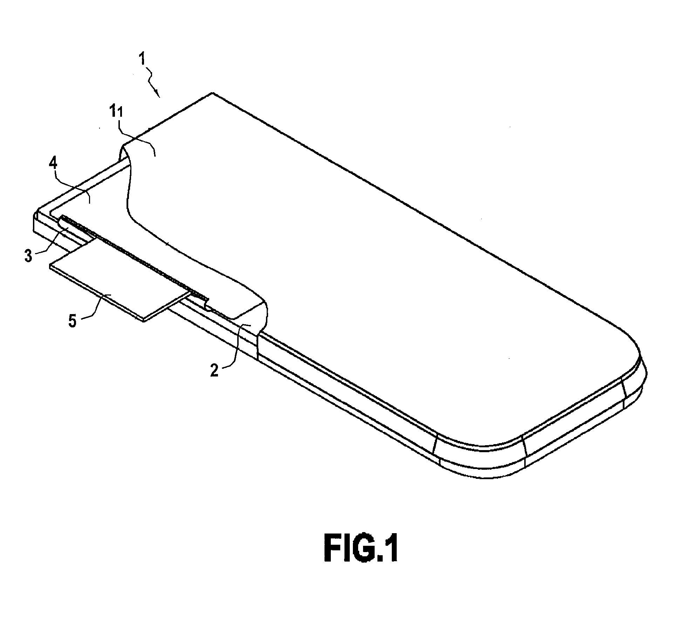

Dans les figures 1 à 3, on a représenté une housse de protection 1 selon l'invention, équipée d'une poche en forme de gaine tubulaire 3 avec des ouvertures 3a opposées débouchant sur les deux bords latéraux 1₂ opposés et bords longitudinaux 1₃ de la partie supérieure 1₁ opposée de la housse de protection.

Les deux ouvertures 3a sont équipées de dispositifs d'ouverture/fermeture à glissière 3b recouverts d'un rabat de protection 3c.

Le bord périphérique de chaque ouverture latérale 3a de la poche 3 est fixé à la housse, par des coutures 3d avec une première couture entre le bord périphérique d'une partie supérieure 3₁ de la poche 3 et un bord longitudinal 1₃ de la partie supérieure 1₁ de la housse et, une deuxième couture 3d entre le bord périphérique d'une partie inférieure 3₂ de la poche et l'extrémité supérieure du rebord latéral 1₂ de la housse recouvrant les flancs 2₂ de l'élément de support ou matelas.

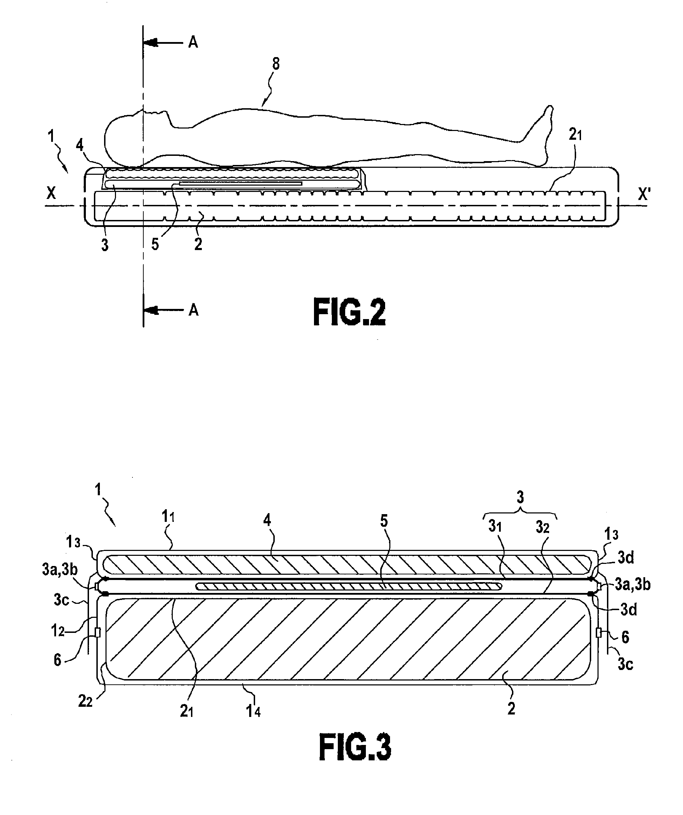

Sur la figure 3, on a représenté un deuxième dispositif de fermeture à glissière 6 qui suit le pourtour périphérique du rebord latéral 1₂ de la housse, de manière à pouvoir retirer le matelas et le rabat de protection 3c protège également ce deuxième dispositif d'ouverture/fermeture à glissière 6.

Ce second dispositif d'ouverture/fermeture à glissière 6 permet de séparer la partie supérieure des rebords latéraux 1₂ de la housse et une partie inférieure 1₄ de la housse.

La poche 3 forme une gaine à travers laquelle peuvent être translatées des cassettes de radiographie 5, d'une ouverture à l'autre, lorsque les dispositifs d'ouverture/fermeture à glissière 3b sont ouverts.

Les tissus constitutifs de la partie supérieure 1₁ et des rebords latéraux 1₂ de la housse de protection sont des tissus synthétiques de type polyester ou polyamide enduits sur au moins la surface externe de la housse d'un polymère polyuréthane présentant des propriétés d'étanchéité, entre autres.

La gaine est constituée d'un matériau textile en polyamide formant un tissu fin de 66 g/m² enduit sur une de ses faces d'une résine fluorée déperlante, et calandré à chaud. Ce tissu présente des propriétés de résistance à la traction et résistance d'au moins 45 daN selon la norme ISO 13934-1 et résistance à la déchirure amorcée selon la norme ISO 13937-1 d'au moins 20N.

De par son calandrage à chaud et son revêtement par une dite couche de résine fluorée, cette toile présente des propriétés de glissement accrues.

Une toile de ce type est commercialisée par la société JEANNE BLANCHIN à CHAMPAGNEUX, 73240 - France sous la référence MELBOURNE 3380

Ce type de tissu souple et fin est classiquement mis en oeuvre pour la confection de voile du type spinnacker et de toile de parachute.

Entre la poche 3 et la partie supérieure 1₁ de la housse sur laquelle repose le patient à radiographier, on intercale un coussin ou élément de support secondaire 4 formé selon la même structure que le matelas principal 2 protégé par ladite housse.

Les différents matériaux mis en oeuvre dans la housse, la poche et l'élément de support secondaire 4 sont transparents aux rayons mis en oeuvre pour la radiographie, notamment les rayons X.

Plus précisément, on met en oeuvre un élément de support principal 2 ou matelas principal 2 constitué de boudins 2₁ disposés transversalement et gonflés d'air, de 10 à 20 cm d'épaisseur.

L'élément de support secondaire 4 est constitué également de boudins 4₁ gonflés à air disposés transversalement mais de plus faible diamètre (à savoir environ 3 cm) s'étendant sensiblement sur toute la largeur du matelas 2 et, dans la direction longitudinale X, X', sur toute la longueur de la poche et limitée à celle-ci.

Les ouvertures 3a de la gaine sont légèrement supérieures à la dimension des cassettes à radiographier standards de plus grands formats, à savoir environ 50 cm.

En revanche, avantageusement, la poche s'étend sur une longueur de 1/2 à 2/3 de la longueur du matelas de manière à couvrir les zones de la partie du corps à radiographier pouvant s'étendre de l'occiput au bassin, quelque soit la morphologie du patient.

On gonfle l'élément de support secondaire 4 avant introduction et évacuation de la cassette par un seul opérateur dans la mesure où les manipulations du patient sont rendues très aisées.

Les essais de radiographies réalisés démontrent une qualité radiographique satisfaisante dépourvue de tout artéfact et la possibilité d'introduction et de retrait de la cassette de radiographie dans la poche par un seul opérateur avec un dérangement minimal du patient à radiographier compatible dans le cas de patients polytraumatisés.

De façon connue, les cellules 2₁, 4₁ des éléments de support principal et éléments de support secondaire 4 sont reliés par un réseau de tuyaux et vannes 7₁ à un dispositif de régulation 7 de pression à l'intérieur des cellules les constituant, le cas échéant, selon les différentes zones du ou desdits matelas, et on régule la pression de l'air dans lesdites cellules de façon à maintenir une homogénéité de la pression d'interface entre le corps du patient et les matelas.

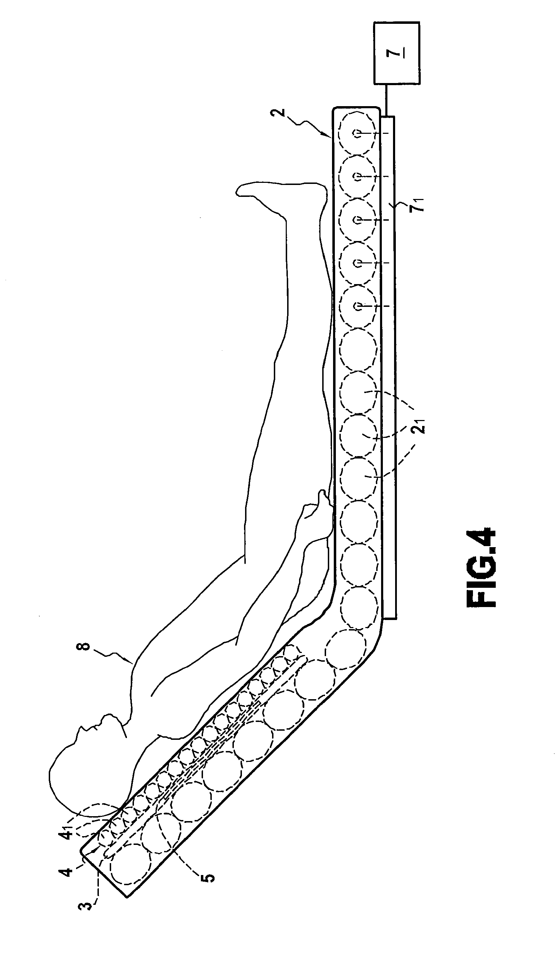

Sur la figure 4, on a représenté une partie seulement du réseau 7₁ de vannes et tuyaux reliant le dispositif de régulation 7 comprenant une pompe de façon à ne pas surcharger le dessin

Avantageusement, la pression dans les cellules dudit élément de support secondaire est identique à celle dans les cellules dudit élément de support principal situé en vis-à-vis dudit élément de support secondaire.

Enfin, comme représenté sur la figure 4, pour éviter un dérangement du patient 8 lié à une surépaisseur du matelas dans la zone correspondant audit élément de support secondaire, on relève et incline à environ 45° le sommier du lit et la zone du matelas correspondant, de façon à ce que la pression d'interface entre le corps et le matelas soit réduite dans la zone dudit élément de support secondaire. Il est ainsi possible de continuer la régulation thérapeutique de la pression à l'intérieur des cellules desdits éléments de support principal et secondaire pendant la radiographie.

## Revendications

1. - Housse de protection (1) d'un élément de support principal (2) du type coussin ou matelas, sur laquelle une partie au moins du corps d'un patient (8) à radiographier est destinée à reposer, ladite housse comprenant au moins une partie supérieure (1₁) sensiblement plane lorsqu'elle est mise en place de manière à recouvrir la surface supérieure (2₁) dudit élément de support principal et des rebords latéraux (1₂), aptes à recouvrir en partie au moins les flancs (2₂) de l'élément de support principal (2), **caractérisée en ce qu'**elle comprend :
- une poche interne (3) souple, s'étendant dessous ladite partie supérieure (1₁) de la housse, apte à contenir une cassette de radiographie (5), ladite poche comprenant au moins une ouverture (3a) permettant l'introduction d'une dite cassette, l'extrémité de la poche formant ouverture débouchant sur et étant fixée à au moins un dit rebord latéral (1₂) de la housse, à proximité du bord longitudinal (1₃) de ladite partie principale de housse, et
- un élément de support secondaire (4) de type matelas ou coussin, moins épais que ledit élément de support principal (2), de préférence d'épaisseur inférieure à 5 cm, solidaire de ladite housse, intercalé entre ladite poche (3) et ladite partie supérieure (1₁) de housse et recouvrant au moins ladite poche.

2. - Housse de protection selon la revendication 1, **caractérisée en ce que** ledit élément de support principal (2) et ledit élément de support secondaire (4) sont constitués chacun par des cellules en forme de boudins (2₁, 4₁) disposées transversalement remplies d'un fluide, de préférence de l'air, les cellules dudit élément de support secondaire présentant une section réduite par rapport à celle dudit élément de support principal, de préférence ladite section réduite présentant une plus grande dimension inférieure à 5 cm.

3. - Housse de protection selon la revendication 2, **caractérisée en ce que** les cellules desdits élément de support principal (2) et élément de support secondaire (4) sont remplies d'air et sont connectées à un dispositif de régulation (7) de la pression à l'intérieur desdites cellules, en fonction de la portance du patient au niveau de différentes zones du corps du patient, la pression à l'intérieur des cellules dudit élément de support secondaire étant sensiblement identique à celle des cellules de la zone dudit élément de support principal en vis-à-vis.

4. - Housse de protection selon l'une des revendication 1 à 3, **caractérisée en ce que** ladite poche est constituée d'un tissu synthétique fin présentant des propriétés mécaniques de résistance à la déchirure et un coefficient de glissement élevés.

5. - Housse de protection selon la revendication 4, **caractérisée en ce que** ledit tissu synthétique est d'un grammage inférieur à 100 g/m², calandré à chaud et, de préférence, dont au moins une face correspondant à la face interne de ladite poche et revêtue d'une couche de résine polymère, de préférence polymère fluoré, lui conférant des propriétés de glissement.

6. - Housse de protection selon l'une des revendications 1 à 5, **caractérisée en ce que** au moins une partie supérieure (3₁) de l'extrémité de la poche formant l'ouverture (3ₐ) est fixée à un dit bord longitudinal (1₃) de ladite partie supérieure (1₁) de housse, et l'autre partie inférieure (3₂) de l'extrémité de la poche formant ladite ouverture (3ₐ) est fixée en haut dudit rebord latéral (1₂) correspondant de la housse.

7. - Housse de protection selon l'une des revendications précédentes, **caractérisée en ce que** ladite poche comporte deux dites ouvertures (3a) opposées, ladite poche formant ainsi une gaine traversant la housse (1) entre les deux dits bords longitudinaux (1₃) opposés et rebords latéraux (1₂) opposés de ladite housse.

8. - Housse de protection selon l'une des revendications précédentes, **caractérisée en ce que** la dimension de la poche dans la direction longitudinale (X, X') dudit élément de support principal (2) permet de positionner une dite cassette (5) en vis-à-vis d'une zone de ladite partie supérieure (1₁) de la housse sur laquelle est destinée à reposer une partie du corps du patient s'étendant depuis l'occiput jusqu'au bassin du patient, de préférence sur une distance de 1/2 à 2/3 de la longueur de ladite housse depuis une de ces extrémités dans la direction longitudinale.

9. - Housse de protection selon l'une des revendications 1 à 8, **caractérisée en ce qu'**elle recouvre un dit élément de support principal (2) de type coussin ou matelas, de préférence disposé respectivement sur un siège ou un lit.

10. - Procédé de radiographie d'une partie au moins du corps d'un patient (7) à l'aide d'une cassette de radiographie (5), au moins la partie du corps du patient (7) à radiographier appuyant sur un dit élément de support principal du type coussin ou matelas, **caractérisé en ce que** l'on insère une dite cassette dans une dite poche d'une housse de protection selon la revendication 9.

11. - Procédé selon la revendication 10, **caractérisé en ce que** l'on met en oeuvre une housse comprenant une poche tubulaire en forme de gaine (3) avec deux ouvertures (3a) débouchant et fixées sur les bords longitudinaux (1₃) opposés et rebords latéraux (1₂) opposés d'une dite housse et on introduit une dite cassette par une dite ouverture, on réalise une radiographie et on évacue ladite cassette par l'ouverture opposée.

12. - Procédé selon l'une des revendications 10 ou 11, **caractérisé en ce qu'**on régule la pression à l'intérieur desdits élément de support principal (2) et élément de support secondaire (4) constitués de cellules en forme de boudins (2₁, 4₁) disposés transversalement et remplis d'air, de façon à homogénéiser la pression d'interface entre le corps du patient et lesdits éléments de support principal et secondaire, en maintenant une pression à l'intérieur des cellules dudit élément de support secondaire sensiblement identique aux cellules dans la zone dudit élément de support principal en vis-à-vis dudit élément de support secondaire.
